# EUROPÄISCHE PATENTSCHRIFT

(11) **EP 4 093 457 B1**
(45) Veröffentlichungstag und Bekanntmachung des Hinweises auf die Patenterteilung: **02.07.2025**
(21) Anmeldenummer: 21703371.1
(22) Anmeldetag: 22.01.2021
(51) Int. Cl.: A61M 1/16, A61M 1/36

(54) **VORRICHTUNG UND VERFAHREN ZUR LUFTFREIEN BEFÜLLUNG EINES FLUIDMANAGEMENTSYSTEMS**
DEVICE AND METHOD OF AIR-FREE PRIMING OF A FLUID MANAGEMENT SYSTEM
DISPOSITIF ET MÉTHODE DE REMPLISSAGE LIBRE D'AIR D'UN SYSTÈME DE GESTION DE FLUIDE

(30) Priorität: 25.01.2020 DE 102020000466
(43) Veröffentlichungstag der Anmeldung: 30.11.2022
(73) Patentinhaber: Fresenius Medical Care Deutschland GmbH, 61352 Bad Homburg (DE)
(72) Erfinder: RÖMMELT, Nico, 97456 Hambach (DE)
(74) Vertreter: Wagner, Andrea
(86) Internationale Anmeldenummer: PCT/EP2021/051418
(87) Internationale Veröffentlichungsnummer: WO 2021/148586

(56) Entgegenhaltungen:
- EP-A1- 2 716 310
- WO-A2-2012/161744
- DE-A1- 102010 025 516
- US-A- 5 702 606
- US-A1- 2014 216 250
- US-A1- 2014 217 020

## Beschreibung

### Technisches Gebiet

Die Erfindung betrifft eine Vorrichtung und ein Verfahren zum luftfreien Befüllen der Hydraulik eines Fluidmanagementsystems, z. B. einer Dialysemaschine.

### Hintergrund

Moderne Dialysemaschinen für die chronische Hämodialyse bereiten die benötigte Dialyselösung online in einem maschinen-internen Hydrauliksystem zu. Dieses maschinen-interne Hydrauliksystem fördert die Dialyselösung in das maschinen-externe extrakorporale Blutleitungssystem, und das verbrauchte Dialysat aus dem Blutleitungssystem wird von diesem Hydrauliksystem aufgenommen und in den Abfluss geleitet.

In der D1 wird ein solches maschinen-externes extrakorporales Blutleitungssystem beschrieben, das zumindest zwei Filtereinrichtungen aufweist. Nach Ankopplung an eine medizinische Blutbehandlungsvorrichtung kann das Blutleitungssystem mit Prozessfluid gefüllt werden.

Die D2 betrifft ebenfalls ein Verfahren zur Befüllung des maschinen-externen extrakorporalen Blutleitungssystems mit Dialysat. Durch Einsatz von Druckpulsen werden Luftblasen auf der Blutseite des Dialysators gelöst und anschließend abtransportiert.

Die D3 betrifft ein System und Verfahren zur Reinigung und Desinfektion eines Behandlungssystems, insbesondere für die Heimdialyse.

Die D4 betrifft die Verwendung eines Antikoagulanz zum Primen eines Schlauchsystems bei der Sammlung und Trennung von Blutkomponenten.

Die D5 betrifft unterschiedliche Aspekte von extrakorporalen Blutschlauchsystemen.

Um die Hygiene des Systems zu gewährleisten ist eine regelmäßige Desinfektion dieses maschinen-internen Hydrauliksystems notwendig.

Insbesondere wenn das Hydrauliksystem vor der Desinfektion mit Luft gefüllt ist, muss bei der Befüllung der Hydraulik mit Flüssigkeit für eine vollständige Entfernung der Luft gesorgt werden, um eine vollständige Benetzung der Oberflächen mit Desinfektionsmittel gewährleisten zu können. Damit wird auch die Wärmeleitung bis zur Dichtung während der Heißdesinfektion gewährleistet, sowie die vollständige Ausspülung des Desinfektionsmittels nach der Desinfektion.

Bei einer einfachen Befüllung der Hydraulik mit einem gleichmäßigen Fluss der Spüllösung, können insbesondere Bauteile der Hydraulik, in denen die Geometrie derselben den Abtransport von Gasblasen durch die Spüllösung behindert, nicht luftfrei gefüllt werden. Gleiches gilt für Kammern und Hohlräume mit einer Geometrie, die dazu führt, dass sich die aufsteigenden Blasen außerhalb der des Flüssigkeitsströmung befinden. Ein Beispiel für eine solche spezielle Geometrie sind Verengungen des Durchflussquerschnitts der Fluidleitungen in der Hydraulik, z.B. eine Kupplungsstelle für den Schlauchsatz eines extrakorporalen Blutkreislaufes, insbesondere der Online-Port.

Die Aufgabe der vorliegenden Erfindung besteht in der Bereitstellung eines Fluidmanagementsystems, insbesondere eines Hydrauliksystems einer Blutbehandlungsmaschine für die Nierenersatztherapie, und ein Verfahren zur Spülung des Fluidmanagementsystems, die eine blasenfreien Füllung der maschineninternen Fluidleitungen gewährleisten.

### Zusammenfassung der Erfindung

Nach der Lehre der vorliegenden Erfindung wird diese Aufgabe gelöst durch eine Vorrichtung nach Anspruch 1 und einem Verfahren nach Anspruch 14. Besondere Ausführungsformen sind Gegenstand der abhängigen Ansprüche.

Die Erfindung betrifft ein Fluidmanagementsystem. Ein solches Fluidmanagementsystem kann z.B. das Hydrauliksystem der Dialysemaschine sein, das zur Herstellung und/oder Förderung der Dialyselösung dient. Das Fluidmanagementsystem weist eine maschinen-interne Fluidleitung (als Maschinenbestandteil) mit einem Fluideingang zur Verbindung mit einer Versorgung mit einer Spülflüssigkeit und einem Fluidausgang zur Verbindung mit einer Leitung zum Abführen der Spülflüssigkeit auf.

Maschinen-intern kann dabei anspruchsgemäß bedeuten, dass die Fluidleitung in einer Ausführungsform vollständig in der Maschine angeordnet ist. Ebenso umfasst eine maschinen-interne Fluidleitung in einer anderen Ausführungsform auch eine Fluidleitung, welche nur teilweise innerhalb der Maschine geführt wird.

In der Fluidleitung ist eine Absperrvorrichtung angeordnet, die die Fluidleitung in einen ersten und einen zweiten Fluidleitungsabschnitt aufteilt.

Der erste Fluidleitungsabschnitt weist ein erstes Fluidförderungsmittel auf, das bei erfindungsgemäßem Betrieb desselben stromauf der Absperrvorrichtung angeordnet ist.

Der zweite Fluidleitungsabschnitt weist ein zweites Fluidförderungsmittel auf, das bei erfindungsgemäßem Betrieb desselben stromab der Absperrvorrichtung angeordnet ist.

Das Fluidmanagementsystem weist eine Steuervorrichtung zum Ansteuern der Absperrvorrichtung, des ersten Fluidförderungsmittels, und des zweiten Fluidförderungsmittels auf, wobei die Steuervorrichtung konfiguriert ist, zur Erhöhung eines Drucks in dem ersten Fluidleitungsabschnitt stromauf der Absperrvorrichtung das erste Fluidförderungsmittel zum Fördern von Flüssigkeit in Richtung der Absperrvorrichtung anzusteuern, und konfiguriert ist, zur Absenkung eines Drucks in einem zweiten Fluidleitungsabschnitt des Fluidleitungssystems stromab der Absperrvorrichtung das zweite Fluidförderungsmittel zum Fördern von Flüssigkeit weg von der Absperrvorrichtung anzusteuern, und wobei die Steuervorrichtung konfiguriert ist, das Absperrmittel bei erhöhtem Druck im ersten Flüssigkeitsabschnitt und bei abgesenktem Druck im zweiten Flüssigkeitsabschnitt wenigstens einmal zu öffnen.

Dabei kann die Steuervorrichtung einen Prozessor, ein Datenspeichermedium und Datenleitungen umfassen. Auf dem Speichermedium kann ein Programmcode hinterlegt sein, bei dessen Ausführungen entsprechende Signale an die jeweiligen Bauteile geschickt werden.

Es ist auch möglich die Absperrvorrichtung wiederholend, z.B. 3 bis 7 mal, abwechselnd zu öffnen und zu schließen. Ein wiederholendes Öffnen und Schließen kann es ermöglichen, dass eine vollständigere oder vollständige Entfernung aller Luftblasen in dem Fluidleitungsabschnitt erreicht wird.

Der Fluidleitungsabschnitt kann zu einem Rezirkulationskreislauf geschlossen werden, wodurch der Verbrauch an Spüllösung verringert wird. Damit die Luft aus dem Rezirkulationskreislauf entfernt werden kann, kann in dem ersten oder bevorzugt in dem zweiten Fluidleitungsabschnitt ein Luftabscheidemittel angeordnet. Das Luftabscheidemittel kann stromab von dem Absperrmittels angeordnet sein. Als solches Luftabscheidemittel kann eine Kammer mit einem Zufluss und einem Abfluss und einer dritten Öffnung im oberen Bereich der Kammer zur Abfuhr von Luft aus der Kammer verwendet werden. Das Luftabscheidemittel kann auch eine Verbindung nach außen mit einer Pumpe sein, wobei die Flüssigkeit mitsamt der Blasen aus dem Fluidabschnitt herausgefördert wird. Als Luftabscheidekammer kann z.B. eine Wassereingangskammer im Zufluss des Fluidmanagementsystems oder eine Kammer im Abfluss des Fluidmanagementsystems verwendet werden. Durch das Rezirkulieren ist man bei höheren Flüssen nicht mehr abhängig von einer hohen Liefermenge durch die Wasserquelle.

Die Fluidleitung, insbesondere der zweite Fluidleitungsabschnitt kann zumindest eine Verengung des Durchgangslumens aufweisen. Luftblasen können insbesondere vor Verengungen des Durchgangslumens der Fluidleitung nicht oder nur schwer passieren.

Solche Engstellen liegen z.B. an den Kupplungsstellen der Hydraulik für den extrakorporalen Blutkreislauf vor. Solche Kupplungsstellen werden verwendet um Dialysat z.B. direkt in das Patientenblut zu fördern. Hier spricht man von einem Substitutionsport. Eine andere Kupplungsstelle, der Spülport, wird als Anschluss des Patientenanschlusses bei der Befüllung und Spülung des Schlauchsets vor Beginn der Behandlung verwendet. Hier ist eine effektive Desinfektion besonders wichtig, da ein direkter Kontakt von Hydraulik und extrakorporalem Blutkreislauf besteht.

Zur optimalen Entfernung der Luftblasen an solchen Engstellen kann das Absperrmittel stromaufwärts dieser Verengung und das zweite Fluidförderungsmittel stromabwärts dieser Verengung angeordnet sein. Bei geschlossenem Absperrmittel entsteht bei Betrieb des zweiten Fluidförderungsmittel ein Unterdruck zwischen Absperrmittel und zweitem Fluidförderungsmittel. Durch diesen Unterdruck vergrößert sich zunächst das Volumen von Gasblasen, die sich in diesem Bereich befinden. Gleichzeitig kann durch Betrieb des ersten Fluidförderungsmittels stromauf der Absperrvorrichtung der Druck in der Fluidleitung vor der geschlossenen Absperrvorrichtung erhöht werden.

Nach Aufbau einer Druckdifferenz zwischen den Fluidleitungsabschnitten stromaufwärts und stromabwärts der Absperrvorrichtung ergibt sich beim Öffnen der Absperrvorrichtung weitgehend ein Druckausgleich und damit stromabwärts der Absperrvorrichtung ein Druckstoß, der die durch den Unterdruck vergrößerten und destabilisierten Gasblasen in kleine Mikroblasen unterteilt. Diese Gasblasen können einfacher die Engstellen im Fluidleitungsabschnitt passieren. Durch den Unterdruck sowie dem Strömungspeak im Fluidleitungsabschnitt stromabwärts der Absperrvorrichtung werden diese kleinen Blasen dann durch die Verengung abgezogen bevor sie wieder zu einer größeren Blase zusammenkommen und können so aus dem Fluidleitungssystem entfernt werden.

Besonders effektiv ist dieser Effekt, wenn die Compliance der Fluidleitung zwischen der Absperrvorrichtung und der Verengung des Durchgangslumens möglichst gering z.B. 0,5 bis 50 cm, bevorzugt 10-30 cm beträgt.

Um zu gewährleisten, dass bei der Öffnung der Absperrvorrichtung eine ausreichende Druckdifferenz vorliegt, kann das Fluidmanagementsystem Druckmessmittel aufweisen und die Steuervorrichtung kann konfiguriert sein, das Öffnen oder das abwechselnde Öffnen und Schließen der Absperrvorrichtung über die durch die Druckmessmittel bestimmten Druckwerte in dem internen Fluidleitungsabschnitt zu steuern. Insbesondere wird dazu ein Druckmessmittel in Fluidverbindung mit dem ersten und ein zweites Druckmessmittel in Fluidverbindung mit dem zweiten Fluidleitungsabschnitt angeordnet.

Eine ausreichende Druckdifferenz ist zum Beispiel erreicht, wenn die Differenz zwischen 1000 und 3000hPa, bevorzugt zwischen 1600 und 2500 hPa liegt.

Das Fluidmanagementsystem kann alternativ oder zusätzlich ein Zeitmessmittel aufweisen. Die Steuervorrichtung kann dann dazu konfiguriert sein, das Öffnen oder das abwechselnde Öffnen und Schließen der Absperrvorrichtung über die durch diese Zeitmessmittel bestimmten Zeiten zu steuern. Das Erreichen einer ausreichenden Druckdifferenz kann bei bekannten Förderraten der Pumpen auch durch die Dauer der Phasen vor der Öffnung der Absperrvorrichtung, bzw. der Dauer der Phase vor der Öffnung der Absperrvorrichtung und die Dauer der Öffnung der Absperrvorrichtung sichergestellt werden.

Das Absperrmittel kann z.B. 1-5 Sekunden, bevorzugt 2 Sekunden geschlossen werden.

Die Öffnung des Absperrmittels kann für 2-6 Sekunden, bevorzugt 4 Sekunden erfolgen.

Außerdem kann die Ventilöffnung schnell erfolgen, so dass der Druckausgleich in einem Zeitintervall zwischen 20 und 500ms erfolgt, vorzugsweise zwischen 20 und 60 ms. So können Gasblasen besonders effektiv weitertransportiert werden.

Die Steuervorrichtung kann alternativ dazu konfiguriert sein, das Öffnen oder das abwechselnde Öffnen und Schließen der Absperrvorrichtung in Abhängigkeit einer bestimmten Anzahl von Arbeitszyklen von zumindest einem Fluidförderungsmittel zu steuern, wenn als Pumpmittel z. B. Membranpumpen oder eine Bilanzkammertaktung verwendet werden.

Das Absperrmittel kann jedes Mittel sein, das geeignet ist die Fluidleitungsabschnitte derart voneinander abzutrennen, dass eine erforderliche Druckdifferenz entsteht. Das Absperrmittel kann bevorzugt ein Ventil sein, z.B. ein elektromagnetisches Ventil oder ein Schlauchquetschventil.

Das Fluidmanagementsystem kann zur Förderung der Spülflüssigkeit jede Art von Fluidförderungsmittel aufweisen, welches geeignet ist den erforderlichen Überdruck, bzw. den erforderlichen Unterdruck aufzubauen. Das Fluidförderungsmittel kann bevorzugt eine Pumpe sein, z.B. peristaltische Pumpen, Membranpumpen oder besonders bevorzugt Zahnradpumpen sein.

Das Fluidmanagementsystem kann z.B. Teil eines Hydrauliksystems einer Blutbehandlungsmaschine für die Nierenersatztherapie sein, z.B. einer Maschine für die Hämodialyse. In dem Hydraulikhydrauliksystem einer solchen Blutbehandlungsmaschine kann z.B. die Entgasungspumpe das erste Fluidförderungsmittel zum Aufbau eines Überdrucks sein und die Flusspumpe die zweite Fluidförderungsmittel zur Erzeugung eines Unterdrucks. Beide Pumpen können Zahnradpumpen sein.

Die Erfindung betrifft auch ein Verfahren zum blasenfreien Befüllen eines erfindungsgemäßen Fluidmanagementsystems mit einer Spülflüssigkeit, wobei das Verfahren besteht in dem Füllen des Systems mit einer Spülflüssigkeit durch Betreiben des ersten und/oder des zweiten Fluidförderungsmittels. Während der Befüllung der Fluidleitung ist das Absperrmittel geöffnet. Nach der Befüllung der Fluidleitung kann das System zunächst für einen bestimmten Zeitraum, z.B. 5-60 Sekunden durch Betreiben des ersten und/oder des zweiten Fluidförderungsmittels gespült werden. Im nächsten Schritt wird die Absperrvorrichtung geschlossen. Zur Erhöhung des Drucks im ersten Fluidleitungsabschnitt und/oder Verminderung des Drucks im zweiten Fluidleitungsabschnitt wird zumindest eines der Fluidförderungsmittels weiterhin betrieben. Die Förderung des Fluids kann auch unterbrochen werden. Nach Schließen des Absperrmittels wird dann zumindest eines betrieben, so dass sich eine Druckdifferenz ausbilden kann. Nachdem sich eine Druckdifferenz ausgebildet hat, wird die Absperrvorrichtung geöffnet.

Das Schließen und Öffnen der Absperrvorrichtung kann mehrmals, vorzugsweise 3 bis 9 mal, erfolgen.

Zu optimalen Entfernung der Luftblasen kann die Öffnung des Absperrmittel bei gleichzeitigem Betrieb zumindest des zweiten Fluidförderungsmittel erfolgen.

Weitere Einzelheiten und Vorteile der Erfindung werden anhand der in den Zeichnungen dargestellten Ausführungsbeispiele näher beschrieben.

### Kurzbeschreibung der Zeichnungen

Figur 1 zeigt eine schematische Darstellung eines erfindungsgemäßen Fluidmanagementsystems.
Figur 2 zeigt ein Ausführungsbeispiel eines hier beschriebenen Verfahrens anhand einer schematischen Darstellung.

### Detaillierte Beschreibung eines Ausführungsbeispiels

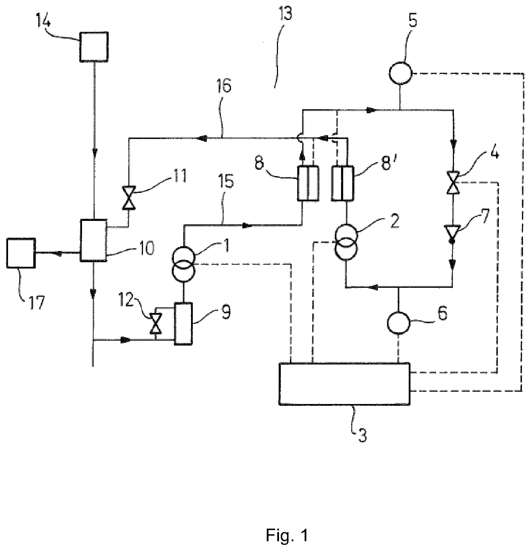

In Abbildung 1 ist in schematischer Weise, exemplarisch für ein Fluidmanagementsystem, ein Teil der Hydraulik einer Dialysemaschine dargestellt.

Die Spülmittelquelle 14 liefert eine Spüllösung zunächst in die Wassereingangskammer 10. Zur Befüllung der Hydraulik wird durch Betrieb der Flusspumpe 2 die Spüllösung in den ersten Fluidabschnitt 15 durch die Entgasungspumpe 1 und die Entgasungskammer 9 in die Frischwasserkammer der linken Bilanzkammer 8 und durch die Absperrvorrichtung 4 in den zweiten Fluidabschnitt 16 geleitet. Die Spüllösung wird dort über die Abwasserseite der rechten Bilanzkammer 8' zurück in die Wassereingangskammer 10 mit einem Belüftungsmittel 17 geleitet. In dem ersten Fluidabschnitt 15 ist ein Druckmessmittel 5 stromaufwärts der Absperrvorrichtung 4 angeordnet. In dem zweiten Fluidabschnitt 16 ist Druckmessmittel 6 stromab der Absperrvorrichtung 4 angeordnet. Außerdem befindet sich im zweiten Fluidabschnitt 16 eine Kupplungsstelle 7 für ein extrakorporales Blutschlauchsystem.

Diese Kupplungsstelle 7 ist an der Maschinenfront angeordnet. Nach Ankopplung an das extrakorporale Blutschlauchsystem kann so eine direkte Lieferung von Dialysat aus der Hydraulik in den extrakorporalen Blutkreislauf erfolgen. Um eine vollständige Desinfektion dieser Kupplungsstelle zu ermöglichen weist dieselbe z.B. eine koaxiale Bauform mit einem Innenrohr und einem koaxial darum angeordneten Außenrohr auf. Das Innenrohr ist gegenüber dem Außenrohr zurückgesetzt. Im Spül- oder Desinfektionsmodus wird das Außenrohr durch eine Klappe nach außen abgedichtet. Im Spül- oder Desinfektionsmodus strömt die Spül-bzw. Desinfektionslösung durch das Innenrohr in das koaxial darum angeordneten Außenrohr und von dort in eine Abflussleitung. Der Abstand zwischenzwischen Innen- und Außenrohr beträgt 6mm. Zudem ist die Kupplungsstelle 7 entlang seiner Längsachse so geneigt, dass der Flüssigkeitsabfluss niedriger angeordnet ist als der Ausgang des Innenrohrs, um eine vollständige Entleerung des Ports von Flüssigkeit zu erleichtern. Deshalb können Luftblasen vor dem zurückgesetzten Innenrohr hängen bleiben und lassen sich nicht ohne weiteres im Spülmodus bei einem laminaren Fluss durch diesen engen Spalt des Außenrohres entgegen der Auftriebskraft in den Abfluss abtransportieren. Gerade diese Engstelle, die die Konnektionsstelle zum Schlauchsystem bildet und damit zu einem eventuell infektiösen Medium, wäre dann nicht vollständig zugänglich für eine Desinfektionslösung. Das luftfreie Befüllen optimiert zudem die Hitzeübertragung durch das flüssige Desinfektionsmittel bis zur Dichtung der Klappe und die vollständige Ausspülung der Desinfektionsmittellösung nach Abschluss der Desinfektion.

Um eine vollständige Entfernung der Luftblasen zu erreichen weist die Dialysemaschine eine Steuereinheit 3 auf. Diese Steuereinheit 3 ist dazu konfiguriert, durch Betrieb der Flusspumpe 2 mit einem kontinuierlichen Fluss die Fluidabschnitte 15 und 16 zu füllen und anschließend zu zirkulieren.

Dann wird bei gleichzeitigem Betrieb der Flusspumpe 2 und der Entgasungspumpe 1 die Absperrvorrichtung 7 geschlossen. Im ersten Fluidabschnitt 15 wird ein Überdruck aufgebaut, im zweiten Fluidabschnitt 16 stellt sich ein Unterdruck ein. Luftblasen, die im zweiten Fluidabschnitt 16 eine Verengung des Durchgangslumens während der Spülung nicht passiert haben, dehnen sich im Unterdruck zunächst aus. Sobald die Druckmessmittel 5 und 6 eine ausreichende Druckdifferenz zwischen erstem 15 und zweitem Fluidabschnitt 16 detektieren, wird die Absperrvorrichtung 4 geöffnet, wodurch ein Druckausgleich erfolgt. Durch den Druckstoß im zweiten Fluidabschnitt 16 werden die Luftblasen in kleinere Gasblasen zerteilt. Diese werden dann sofort durch die Entgasungspumpe durch die Verengung gefördert und gelangen dann in die Wassereingangskammer 10, wo sie in die Atmosphäre abgeleitet werden. Wenn der Fluidlevel in der Wassereingangskammer unter einen festgelegten Wert fällt, wird sie mit Spüllösung aufgefüllt.

Soll eine Entgasung während Phasen mit hohem Fluss vermieden werden, kann durch Öffnen des Ventils 12 die Entgasungsdrossel 9 umgangen werden.

Der Vorgang des abwechselnden Schließens und Öffnens der Absperrvorrichtung 7 kann mehrmals, z.B. 7 mal, wiederholt werden.

Anschließend werden die Fluidfördermittel 1 und 2 gestoppt, das System belüftet und die Ventile geschlossen.

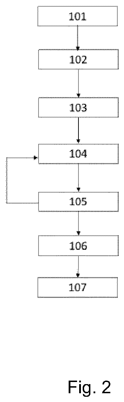

In Abbildung 2 ist ein Flussdiagramm einer Ausführungsform des erfindungsgemäßen Verfahrens dargestellt.

Zum Beginn der Dialysebehandlung wird das Außenrohr der koaxialaufgebauten Kupplungsstelle der Dialysemaschine für das extrakorporale Blutschlauchsystem und die davon abführende Fluidleitung entleert und ist damit mit Luft gefüllt.

In einem ersten Schritt 101 des Füllungsverfahrens werden zunächst die in Abbildung 1 dargestellte Wassereingangskammer 10 und jeweils ein Abteil der Bilanzkammern mit Wasser gefüllt.

In einem zweiten Verfahrensschritt 102 wird die Spüllösung in dem in Abbildung 1 gezeigten Fluidkreislauf zirkuliert. Die Ventile 4, 11 und 12 sind geöffnet. Dieser Verfahrensschritt dauert circa 5 Sekunden.

In einem dritten Verfahrensschritt 103 wird die Kupplungsstelle mit einem kontinuierlichen Fluss gespült. Dieser Verfahrensschritt 103 dauert circa 5 Sekunden.

In einem vierten Verfahrensschritt 104 wird das Ventil 4 geschlossen und durch Betrieb der Entgasungspumpe 1 wird stromauf des Ventils ein Überdruck von über 1800 hPa aufgebaut. Durch Betrieb der Flusspumpe 2 wird stromab des Ventils 4 ein Unterdruck von weniger als -400 hPa aufgebaut. Dieser Verfahrensschritt dauert circa 2 Sekunden.

In einem fünften Verfahrensschritt 105 wird die Druckdifferenz abgebaut, indem Ventil 4 für circa 4 Sekunden geöffnet wird.

Zur vollständigen Entfernung der Luft werden dann die Verfahrensschritte 104 und 105 bis zu sieben Mal wiederholt.

In einem sechsten Verfahrensschritt 106 werden die Pumpen 1 und 2 gestoppt und das System belüftet.

In einem siebten Verfahrensschritt 107 werden die verwendeten Ventile geschlossen.

### Bezugszeichenliste:

Erste Pumpe 1
Zweite Pumpe 2
Steuereinheit 3
Absperrmittel 4
Erstes Druckmessmittel 5
Zweites Druckmessmittel 6
Kupplungsstelle für extrakorporales Blutschlauchsystem 7
Bilanzkammern 8
Entgasungskammer 9
Wassereingangskammer 10
Ventil 11
Ventil 12
Fluidmanagementsystem 13
Spülmittelquelle 14
Erster Fluidleitungsabschnitt 15
Zweiter Fluidleitungsabschnitt 16
Belüftungsmittel 17

## Patentansprüche

1. Fluidmanagementsystem (13) aufweisend
eine maschinen-interne Fluidleitung verbindbar mit einem Fluideingang zur Versorgung mit einer Spülflüssigkeit und einem Fluidausgang zum Abführen der Spülflüssigkeit,
eine Absperrvorrichtung (4) in der Fluidleitung, die die Fluidleitung in einen ersten und einen zweiten Fluidleitungsabschnitt (15,16) aufteilt,
einem ersten Fluidförderungsmittel (1) in dem ersten Fluidleitungsabschnitt (15) bei Betrieb des ersten Fluidförderungsmittels (1) angeordnet stromauf der Absperrvorrichtung,
einem zweiten Fluidförderungsmittel (2) in dem Fluidleitungsabschnitt (16) bei Betrieb des zweiten Fluidförderungsmittels (2) angeordnet stromab der Absperrvorrichtung (4), und
einer Steuervorrichtung (3) zum Ansteuern der Absperrvorrichtung (4), des ersten Fluidförderungsmittels (1), und des zweiten Fluidförderungsmittels (2),
wobei die Steuervorrichtung (3) konfiguriert ist, zur Erhöhung eines Drucks in dem ersten Fluidleitungsabschnitt (15) relativ zu einem Druck in dem zweiten Fluidleitungsabschnitt (16) das erste Fluidförderungsmittel (1) zum Fördern von Flüssigkeit in Richtung der Absperrvorrichtung (4) anzusteuern, und konfiguriert ist, zur Absenkung eines Drucks in einem zweiten des Fluidleitungsabschnitt (16) relativ zu dem Druck in dem ersten Fluidleitungsabschnitts stromab der Absperrvorrichtung (4) die zweite Fluidförderungsmittel (2) zum Fördern von Flüssigkeit weg von der Absperrvorrichtung (4) anzusteuern, und
wobei die Steuervorrichtung (3) konfiguriert ist, das Absperrmittel (4) bei erhöhtem Druck im ersten Flüssigkeitsabschnitt (15) und bei abgesenktem Druck im zweiten Flüssigkeitsabschnitt (16) wenigstens einmal zu öffnen.

2. Fluidmanagementsystem (13) nach Anspruch 1, wobei die Steuervorrichtung (3) dazu konfiguriert ist das Absperrmittel (4) wiederholend zum Aufbau einer Druckdifferenz zwischen dem ersten und zweiten Fluidleitungsabschnitt (15, 16) zu schließen und zum Ausgleich der Druckdifferenz zu öffnen.

3. Fluidmanagementsystem (13) nach Anspruch 1 oder 2, wobei der erste und der zweite Fluidleitungabschnitt (15, 16) zu einem Rezirkulationskreislauf geschlossen sind und ein Luftabscheidemittel aufweisen.

4. Fluidmanagementsystem (13) nach einem der vorangehenden Ansprüche, wobei einer der Fluidleitungsabschnitte (15, 16), bevorzugt der zweite Fluidleitungsabschnitt (16) zumindest eine Verengung des Durchgangslumens aufweist und das Absperrmittel (4) stromaufwärts und das zweite Pumpmittel (2) stromabwärts der Verengung angeordnet sind.

5. Fluidmanagementsystem (13) nach Anspruch 4, wobei das Absperrmittel (4) im Fluidleitungsabschnitt 0,5 bis 50 cm, bevorzugt 10-30 cm vor der Verengung des Durchgangslumens angeordnet ist.

6. Fluidmanagementsystem (13) nach Anspruch 4, wobei die Verengung des Durchgangslumens in einer Anschlussstelle für den extrakorporalen Blutkreislauf angeordnet ist.

7. Fluidmanagementsystem (13) nach einem der vorhergehenden Ansprüche, wobei das Fluidmanagment-System zumindest in einem von dem ersten und dem zweiten Fluidleitungsabschnitt (15, 16) jeweils ein Druckmessmittel (5,6) aufweist und die Steuervorrichtung (3) dazu konfiguriert ist, das Öffnen oder das abwechselnde Öffnen und Schließen der Absperrvorrichtung (4) über die durch die Druckmessmittel (5,6) bestimmten Druckwerte zu steuern.

8. Fluidmanagementsystem (13) nach Anspruch 7, wobei ein Öffnen des Absperrmittels (4) erfolgt, wenn sich eine Druckdifferenz zwischen stromauf- und stromabwärtigem Fluidleitungsabschnitt (15, 16) von 1000 bis 3000hPa, bevorzugt von 1600 bis 2500 hPa eingestellt hat.

9. Fluidmanagementsystem (13) nach einem der vorangehenden Ansprüche, wobei Fluidmanagment-System ein Zeitmessmittel aufweist und die Steuervorrichtung (3) dazu konfiguriert ist, das Öffnen oder das abwechselnde Öffnen und Schließen der Absperrvorrichtung (4) über die durch die Zeitmessmittel bestimmten Zeiten zu steuern.

10. Fluidmanagementsystem (13) nach einem der vorangehenden Ansprüche, wobei die Steuervorrichtung (3) dazu konfiguriert ist, das Öffnen oder das abwechselnde Öffnen und Schließen der Absperrvorrichtung (4) in Abhängigkeit eines Arbeitszyklus von zumindest einer der Fluidförderungsmittel (1, 2) zu steuern.

11. Fluidmanagementsystem (13) nach einem der vorangehenden Ansprüche, wobei das Absperrmittel (4) ein Ventil, z. B. ein elektromagnetisches Ventil oder ein Schlauchquetschventil ist.

12. Fluidmanagementsystem (13) nach einem der vorangehenden Ansprüche, wobei die Fluidförderungmittel Pumpen sind, z.B. peristaltische Pumpen oder bevorzugt Zahnradpumpen sind.

13. Fluidmanagementsystem (13) nach einem der vorangehenden Ansprüche wobei das Fluidmanagementsystem Teil eines Hydrauliksystems einer Blutbehandlungsmaschine für die Nierenersatztherapie ist.

14. Verfahren zum Spülen eines maschinen-internen Fluidmanagementsystems, bevorzugt nach einem der Ansprüche 1 bis 13, wobei das Verfahren darin besteht
- A) Befüllen einer Fluidleitung des Fluidmanagementsystems mit einer Spülflüssigkeit durch Betreiben zumindest eines Fluidförderungsmittels (1, 2), während die Absperrvorrichtung (4) geöffnet ist,
- anschließend
- B) Schließen der Absperrvorrichtung (4) und
- C) Aufbau einer Druckdifferenz zwischen erstem und zweitem Fluidleitungsabschnitt (15, 16) durch Betreiben von mindest eines der Fluidförderungsmittel
- D) Öffnen der Absperrvorrichtung. (4)

15. Verfahren nach Anspruch 14, wobei die Verfahrensschritte B) bis D) mehrmals wiederholt werde, bevorzugt 3-7 mal.

16. Verfahren nach einem der Ansprüche 14 oder 15, wobei bei Öffnen des Absperrmittels (4) zumindest das zweite Fluidförderungsmittel (2) in Betrieb ist.

## Claims

1. Fluid management system (13) comprising
a machine-internal fluid line which can be connected to a fluid inlet for supplying a flushing fluid and a fluid outlet for discharging the flushing fluid,
a shut-off device (4) in the fluid line, which divides the fluid line into a first and a second fluid line portion (15, 16),
a first fluid conveying means (1) in the first fluid line portion (15) arranged upstream of the shut-off device during operation of the first fluid conveying means (1), a second fluid conveying means (2) in the fluid line portion (16) arranged downstream of the shut-off device (4) during operation of the second fluid conveying means (2), and
a control device (3) for actuating the shut-off device (4), the first fluid conveying means (1) and the second fluid conveying means (2),
wherein the control device (3) is configured, in order to increase a pressure in the first fluid line portion (4) relative to a pressure in the second fluid line portion (16), to actuate the first fluid conveying means (1) for conveying fluid in the direction of the shut-off device (15), and is configured, in order to lower a pressure in a second fluid line portion (4) relative to the pressure in the first fluid line portion downstream of the shut-off device (4), to actuate the second fluid conveying means (2) for conveying fluid away from the shut-off device (16), and
wherein the control device (3) is configured to open the shut-off agent (4) at least once at increased pressure in the first fluid portion (15) and at lowered pressure in the second fluid portion (16).

2. Fluid management system (13) according to Claim 1, wherein the control device (3) is configured to repeatedly close the shut-off means (4) in order to build up a pressure difference between the first and second fluid line portion (15, 16) and to open it in order to equalize the pressure difference.

3. Fluid management system (13) according to Claim 1 or 2, wherein the first and the second fluid line portion (15, 16) are closed to form a recirculation circuit and have an air separator means.

4. Fluid management system (13) according to one of the preceding claims, wherein one of the fluid line portions (15, 16), preferably the second fluid line portion (16) has at least one constriction of the passage lumen and the shut-off means (4) is arranged upstream of and the second pumping means (2) is arranged downstream of the constriction.

5. Fluid management system (13) according to Claim 4, wherein the shut-off means (4) is arranged in the fluid line portion upstream of the constriction of the passage lumen by from 0.5 to 50 cm, preferably 10-30 cm.

6. Fluid management system (13) according to Claim 4, wherein the constriction of the passage lumen is arranged at a connection point for the extracorporeal blood circulation.

7. Fluid management system (13) according to one of the preceding claims, wherein the fluid management system has, at least in one of the first and the second fluid line portions (15, 16), in each case a pressure measuring means (5, 6), and the control device (3) is configured to control the opening or alternately opening and closing of the shut-off device (4) via the pressure values determined by the pressure measuring means (5, 6).

8. Fluid management system (13) according to Claim 7, wherein opening of the shut-off means (4) takes place when a pressure difference between the upstream and downstream fluid line portion (15, 16) of from 1000 to 3000 hPa, preferably of from 1600 to 2500 hPa has been set.

9. Fluid management system (13) according to one of the preceding claims, wherein the fluid management system has a time measuring means, and the control device (3) is configured to control the opening or the alternating opening and closing of the shut-off device (4) via the times determined by the time measuring means.

10. Fluid management system (13) according to one of the preceding claims, wherein the control device (3) is configured to control the opening or the alternating opening and closing of the shut-off device (4) in a manner dependent on a working cycle of at least one of the fluid conveying means (1, 2).

11. Fluid management system (13) according to one of the preceding claims, wherein the shut-off means (4) is a valve, e.g. an electromagnetic valve or a constriction-hose valve.

12. Fluid management system (13) according to one of the preceding claims, wherein the fluid conveying means are pumps, e.g. peristaltic pumps or preferably gear pumps.

13. Fluid management system (13) according to one of the preceding claims, wherein the fluid management system is part of a hydraulic system of a blood treatment machine for renal replacement therapy.

14. Method for flushing a machine-internal fluid management system, preferably according to one of Claims 1 to 13, wherein the method consists of
- A) filling a fluid line of the fluid management system with a flushing fluid by operating at least one fluid conveying means (1, 2) while the shut-off device (4) is open,
- then
- B) closing the shut-off device (4), and
- C) building up a pressure difference between the first and second fluid line portions (15, 16) by operating at least one of the fluid conveying means,
- D) opening the shut-off device (4).

15. Method according to Claim 14, wherein the method steps B) to D) are repeated several times, preferably 3-7 times.

16. Method according to either of Claims 14 or 15, wherein at least the second fluid conveying means (2) is in operation when the shut-off means (4) is opened.

## Revendications

1. Système (13) de gestion de fluide comprenant
une conduite de fluide interne à la machine aptes à être raccordée à une entrée de fluide pour l'alimentation en liquide de rinçage et à une sortie de fluide pour l'évacuation du liquide de rinçage,
un dispositif d'arrêt (4) dans la conduite de fluide, qui divise la conduite de fluide en une première et une deuxième sections (15, 16) de conduite de fluide,
un premier moyen (1) de transport de fluide dans la première section (15) de conduite de fluide agencé, lors du fonctionnement, en amont du dispositif d'arrêt du premier moyen (1) de transport de fluide,
un deuxième moyen (2) de transport de fluide dans la section (16) de conduite de fluide, agencé, lors du fonctionnement, en aval du dispositif d'arrêt (4) du deuxième moyen (2) de transport de fluide, et
un dispositif de commande (3) pour commander le dispositif d'arrêt (4), le premier moyen (1) de transport de fluide et le deuxième moyen (2) de transport de fluide, le dispositif de commande (3) étant conçu pour commander le premier moyen (1) de transport de fluide de façon à transporter du liquide en direction du dispositif d'arrêt (4) afin d'augmenter une pression dans la première section (15) de conduite de fluide par rapport à une pression dans la deuxième section (16) de conduite de fluide, et configuré pour commander le deuxième moyen (2) de transport de fluide de façon à transporter le liquide en éloignement du dispositif d'arrêt (4) afin d'abaisser une pression dans une deuxième section (16) de conduite de fluide par rapport à la pression dans la première section de conduite de fluide en aval du dispositif d'arrêt (4), et
le dispositif de commande (3) étant configuré pour ouvrir ledit moyen d'arrêt (4) au moins une fois lorsque la pression est élevée dans la première section de liquide (15) et lorsque la pression est abaissée dans la deuxième section de liquide (16).

2. Système (13) de gestion de fluide selon la revendication 1, dans lequel le dispositif de commande (3) est configuré pour fermer de manière répétée le moyen de fermeture (4) afin d'établir une différence de pression entre les première et deuxième sections (15, 16) de conduite de fluide, et pour l'ouvrir afin de compenser la différence de pression.

3. Système de gestion de fluide (13) selon la revendication 1 ou la revendication 2, dans lequel les première et deuxième sections (15, 16) de conduite de fluide sont fermées de façon à former un circuit de recirculation et comprennent un moyen de séparation de l'air.

4. Système (13) de gestion de fluide selon l'une des revendications précédentes, dans lequel l'une des sections (15, 16) de conduite de fluide, de préférence la deuxième section (16) de conduite de fluide, comprend au moins un rétrécissement de la lumière de passage, et le moyen d'arrêt (4) est agencé en amont et le deuxième moyen de pompage (2) est agencé en aval du rétrécissement.

5. Système (13) de gestion de fluide selon la revendication 4, dans lequel le moyen d'arrêt (4) est agencé dans la section de conduite de fluide de 0,5cm à 50cm, de préférence de 10cm à 30cm avant le rétrécissement de la lumière de passage.

6. Système (13) de gestion de fluide selon la revendication 4, dans lequel le rétrécissement de la lumière de passage est agencé dans un point de raccordement pour la circulation sanguine extracorporelle.

7. Système (13) de gestion de fluide selon l'une des revendications précédentes, le système de gestion de fluide comprenant au moins un moyen de mesure de pression (5, 6) dans chacune des première et deuxième sections (15, 16) de conduite de fluide, et le dispositif de commande (3) étant configuré pour commander l'ouverture, ou l'ouverture et la fermeture alternées, du dispositif d'arrêt (4) en fonction des valeurs de pression déterminées par les moyens de mesure de pression (5, 6).

8. Système (13) de gestion de fluide selon la revendication 7, dans lequel l'ouverture du dispositif d'arrêt (4) se produit lorsqu'une différence de pression entre les sections de conduite de fluide en amont et en aval (15, 16) s'est établie entre 1000hPa et 3000hPa, de préférence entre 1600hPa et 2500hPa.

9. Système (13) de gestion de fluide selon l'une des revendications précédentes, le système de gestion de fluide comprenant un moyen de chronométrage et le dispositif de commande (3) étant configuré pour commander l'ouverture, ou l'ouverture et la fermeture alternées, du dispositif d'arrêt (4) pendant les durées déterminées par le moyen de chronométrage.

10. Système (13) de gestion de fluide selon l'une des revendications précédentes, dans lequel le dispositif de commande (3) est configuré pour commander l'ouverture ou l'ouverture et la fermeture alternées du dispositif d'arrêt (4) en fonction d'un cycle de travail d'au moins l'un des moyens de transport de fluide (1, 2).

11. Système (13) de gestion de fluide selon l'une des revendications précédentes, dans lequel le moyen d'arrêt (4) est une vanne, par exemple une vanne électromagnétique ou une vanne à manchon.

12. Système (13) de gestion de fluide selon l'une des revendications précédentes, dans lequel les moyens de transport de fluide sont des pompes, par exemple des pompes péristaltiques ou de préférence des pompes à engrenages.

13. Système (13) de gestion de fluide selon l'une des revendications précédentes, dans lequel le système de gestion de fluide fait partie d'un système hydraulique d'une machine de traitement du sang pour la thérapie de remplacement rénal.

14. Procédé de rinçage d'un système de gestion de fluide interne à une machine, de préférence selon l'une des revendications 1 à 13, le procédé consistant
- A) à remplir une conduite de fluide du système de gestion de fluide avec un liquide de rinçage en actionnant au moins un moyen de transport de fluide (1, 2) pendant que le dispositif d'arrêt (4) est ouvert,
- puis
- B) fermeture du dispositif d'arrêt (4), et
- C) établissement d'une différence de pression entre la première et la deuxième section (15, 16) de conduite de fluide par l'actionnement d'au moins un des moyens de transport de fluide,
- D) ouverture du dispositif d'arrêt (4).

15. Procédé selon la revendication 14, dans lequel les étapes B) à D) sont répétées plusieurs fois, de préférence 3 à 7 fois.

16. Procédé selon l'une des revendications 14 ou 15, dans lequel, lors de l'ouverture du dispositif d'arrêt (4), au moins le deuxième moyen (2) de transport de fluide est en fonctionnement.
